# EUROPEAN PATENT APPLICATION

(11) **EP 4 670 615 A1**
(43) Date of publication of application: **31.12.2025**
(21) Application number: 25184921.2
(22) Date of filing: 24.06.2025
(51) Int. Cl.: A61B 3/10, A61B 3/103, A61B 3/107, A61B 3/12, A61B 3/135, A61B 3/15, A61B 3/16, A61B 3/00, A61B 3/117

(54) **OPHTHALMIC APPARATUS AND OPHTHALMIC APPARATUS CONTROL PROGRAM**

(30) Priority: 25.06.2024 JP 2024102335
(71) Applicant: NIDEK CO., LTD., Gamagori, Aichi (JP)
(72) Inventor: SUZUKI, Kunio, Gamagori, Aichi (JP)
(74) Representative: Hoefer & Partner Patentanwälte mbB

(57) **Abstract**

An ophthalmic apparatus for examining a subject eye includes an examination means configured to examine the subject eye, an approach unit provided with the examination means, a detection means configured to detect an approach of the approach unit approaching an examinee, and a control means configured to perform, when the approach is detected by the detection means, automatic alignment control different from that performed when the approach is not detected.

## Description

### TECHNICAL FIELD

The present disclosure relates to an ophthalmic apparatus for examining a subject eye, and an ophthalmic apparatus control program to be executed in the ophthalmic apparatus.

### BACKGROUND ART

Examples of related ophthalmic apparatuses include an eye refractive power measurement apparatus, a corneal curvature measurement apparatus, an intraocular pressure measurement apparatus, a fundus camera, an optical coherence tomography (OCT), and a scanning laser ophthalmoscope (SLO). In these ophthalmic apparatuses, it is common to move an examination unit in up, down, left, right, front, and rear directions with respect to a subject eye and align the examination unit to a predetermined position with respect to a subject eye. In addition, in order to prevent a nozzle or the like provided in an examination unit from coming into contact with the subject eye, there has been proposed an apparatus that includes a contact sensor or the like for detecting the contact and performs an avoidance operation when the subject eye and the examination unit come into contact with each other (see JPH07-255677A).

However, depending on a shape of a face of an examinee, the examination may be performed only in a state where the examination unit is in contact with the examinee. In this case, in order for an examiner to perform alignment in the related ophthalmic apparatus, the examiner needs to be familiar with the operation.

### SUMMARY OF INVENTION

A technical object of the present disclosure is to provide an ophthalmic apparatus and an ophthalmic apparatus control program that enable an examiner to easily perform alignment while ensuring safety.
(1) An ophthalmic apparatus for examining a subject eye, the ophthalmic apparatus including:
   an examination means configured to examine the subject eye;
   an approach unit provided with the examination means, the approach unit approaching an examinee;
   a detection means configured to detect an approach of the approach unit to the examinee; and
   a control means configured to perform, when the approach is detected by the detection means, automatic alignment control different from that performed when the approach is not detected.
(2) The ophthalmic apparatus according to the above-described (1), in which
   in a case where the approach is detected, the control means continues automatic alignment under the automatic alignment control.
(3) The ophthalmic apparatus according to the above-described (1) or (2), in which
   when the approach is detected, the control means performs the automatic alignment control to restrict a movement of the approach unit.
(4) The ophthalmic apparatus according to any one of the above-described (1) to (3), in which
   when the approach is detected, the control means performs the automatic alignment control to reduce an automatic alignment range as compared with when the approach is not detected.
(5) The ophthalmic apparatus according to any one of the above-described (1) to (4), in which
   when the approach is detected, the control means performs the automatic alignment control to reduce a movement range of the approach unit as compared with when the approach is not detected.
(6) The ophthalmic apparatus according to any one of the above-described (1) to (5), in which
   when the approach is detected, the control means performs the automatic alignment control to reduce a moving speed of the approach unit as compared with when the approach is not detected.
(7) The ophthalmic apparatus according to any one of the above-described (1) to (6), in which
   the ophthalmic apparatus is a non-contact tonometer including a fluid discharge means configured to discharge a fluid to a cornea of the subject eye via a nozzle unit, and
   the approach unit is the nozzle unit.
(8) An ophthalmic apparatus control program of an ophthalmic apparatus for examining a subject eye, the ophthalmic apparatus including an examination means configured to examine the subject eye, an approach unit provided with the examination means, a detection means configured to detect an approach of the approach unit to an examinee, and a control means,
   the ophthalmic apparatus control program including instructions which, when executed by a control means of the ophthalmic apparatus, cause the ophthalmic apparatus to perform:
   a detection step of detecting an approach of the approach unit approaching an examinee by the detection means; and
   a control step of performing, when the approach is detected in the detection step, automatic alignment control different from that performed when the approach is not detected.

### BRIEF DESCRIPTION OF DRAWINGS

Exemplary embodiment(s) of the present invention will be described in detail based on the following figures, wherein:
FIG. 1 is a diagram illustrating an external configuration of an ophthalmic apparatus;
FIG. 2 is a diagram illustrating an internal configuration of the ophthalmic apparatus;
FIG. 3 is a diagram illustrating a configuration of a measurement optical system of the ophthalmic apparatus;
FIG. 4 is a flowchart illustrating examination processing executed by the ophthalmic apparatus;
FIG. 5 illustrates an example of an anterior segment image displayed on a display unit of the ophthalmic apparatus;
FIG. 6 illustrates an example of the anterior segment image displayed on the display unit of the ophthalmic apparatus;
FIG. 7 illustrates an example of the anterior segment image displayed on the display unit of the ophthalmic apparatus;
FIG. 8 illustrates an example of a notification displayed on the display unit of the ophthalmic apparatus;
FIG. 9 illustrates an example of a notification displayed on the display unit of the ophthalmic apparatus; and
FIG. 10 illustrates an example of a notification displayed on the display unit of the ophthalmic apparatus.

### DESCRIPTION OF EMBODIMENTS

An ophthalmic apparatus according to the present disclosure will be described. The ophthalmic apparatus (for example, an ophthalmic apparatus 1) according to the present disclosure is an apparatus for examining a subject eye. Examples of the ophthalmic apparatus include an intraocular pressure measurement apparatus, an eye refractive power measurement apparatus, a corneal curvature measurement apparatus, a corneal shape measurement apparatus, an axial length measurement apparatus, a fundus camera, an OCT, and an SLO. The ophthalmic apparatus according to the present disclosure includes an examination unit (for example, examination unit 100), an approach unit (for example, nozzle unit 205), a detection unit (for example, detection unit 250), and a control unit (for example, control unit 80). The examination unit examines the subject eye. The approach unit is provided with the examination unit. The approach unit approaches an examinee. The detection unit detects an approach of the approach unit to the examinee. The detection unit may be provided at the approach unit, for example. The detection unit may be, for example, a contact sensor provided at the approach unit, and may detect the approach by detecting contact between the approach unit and the examinee. The detection unit may be, for example, a capacitance sensor provided at the approach unit, and may detect the approach to (or contact with) the examinee. When the approach is detected by the detection unit, the control unit performs automatic alignment control different from that performed when the approach is not detected.

With the above configuration, when the approach is detected by the detection unit, the ophthalmic apparatus according to the present disclosure performs the automatic alignment control different from that performed when the approach is not detected. The related ophthalmic apparatus performs, for example, an avoidance operation when detecting the approach. However, by performing the automatic alignment control different from that performed when the approach is not detected, the control unit can continue the automatic alignment control even after the detection unit detects the approach. Therefore, an examiner can easily perform alignment.

The automatic alignment control in the present disclosure is control for automatically aligning the examination unit to a position where the measurement of the subject eye can be performed. Examples of the automatic alignment control include rough adjustment for roughly and quickly aligning the examination unit, fine adjustment for fine and precise alignment, and tracking for tracking an eye movement. When the examiner manually performs alignment, control for driving a drive unit in accordance with an operation signal or the like is referred to as manual alignment control.

When the approach is detected, the control unit continues the automatic alignment under the automatic alignment control. The related ophthalmic apparatus stops the automatic alignment when detecting the approach. In this case, the examiner needs to manually perform the alignment. By continuing the automatic alignment, a case in which the examiner needs to manually perform the alignment is reduced. Therefore, the burden on the examiner is reduced.

When the approach is detected, the control unit may perform the automatic alignment control to restrict a movement of the approach unit. Accordingly, an unexpected movement of the approach unit is prevented, thereby improving operability.

For example, when the approach is detected, the control unit may restrict the movement of the approach unit by performing the automatic alignment control to reduce an automatic alignment range as compared with when the approach is not detected. The automatic alignment range is a range within which the control unit performs the automatic alignment. Accordingly, it is possible to prevent a large distance movement of the approach unit when the approach unit approaches the examinee, thereby improving the operability.

In addition, for example, when the approach is detected, the control unit may restrict the movement of the approach unit by performing the automatic alignment control to reduce a movement range of the approach unit as compared with when the approach is not detected. Accordingly, the movement of the approach unit to an unexpected position during the detection of the approach of the approach unit to the examinee can be reduced, and the operability is improved.

When the approach is detected, the control unit performs the automatic alignment control to reduce a moving speed of the approach unit as compared with when the approach is not detected. Accordingly, the control unit can prevent a sudden movement of the approach unit, thereby improving the operability.

The ophthalmic apparatus may be a non-contact tonometer including a fluid discharge means configured to discharge a fluid to a cornea of the subject eye via a nozzle unit, and in this case, the approach unit is the nozzle unit. In intraocular pressure measurement using the non-contact tonometer, it is necessary to accurately detect deformation of the cornea of the subject eye by blowing a fluid to the cornea. Therefore, an alignment completion range is smaller than that of other ophthalmic apparatuses. Manual alignment by the examiner particularly imposes a burden on the examiner. Therefore, by applying the present disclosure to a tonometer, even when the approach unit approaches the examinee, the control unit performs alignment control, which leads to a reduction in burden on the examiner in particular.

Hereinafter, the ophthalmic apparatus according to the present embodiment will be described with reference to the drawings. Although the intraocular pressure measurement apparatus will be described as an example of the ophthalmic apparatus, the present invention is also applicable to other ophthalmic apparatuses such as an eye refractive power measurement apparatus, a corneal curvature measurement apparatus, a corneal shape measurement apparatus, an axial length measurement apparatus, a fundus camera, an OCT, or an SLO.

The ophthalmic apparatus according to the present embodiment measures, for example, an intraocular pressure of the subject eye in a non-contact manner. For example, the ophthalmic apparatus according to the present embodiment may perform measurement for each eye, or may perform measurement for both eyes simultaneously. The ophthalmic apparatus may measure only one of right and left subject eyes.

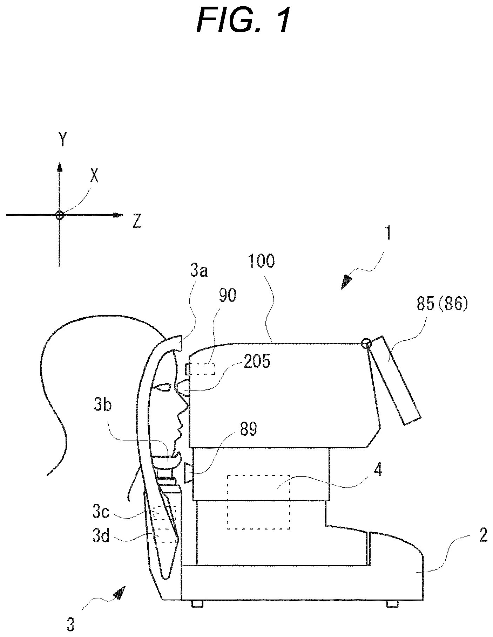

As illustrated in FIG. 1, the ophthalmic apparatus 1 according to the present embodiment includes the examination unit 100, the detection unit 250, the control unit 80, and the like. Hereinafter, each configuration will be described.

The examination unit 100 is an examination means that performs examination (measurement, imaging, or the like) of the subject eye. The examination unit 100 includes, for example, a fluid ejection unit and a measurement optical system for measuring the intraocular pressure of the subject eye. Of course, the examination unit 100 may include an optical system for measuring eye refractive power, a corneal shape, and the like. The examination unit 100 may include an optical system or the like for imaging the anterior segment, the fundus, and the like of the subject eye.

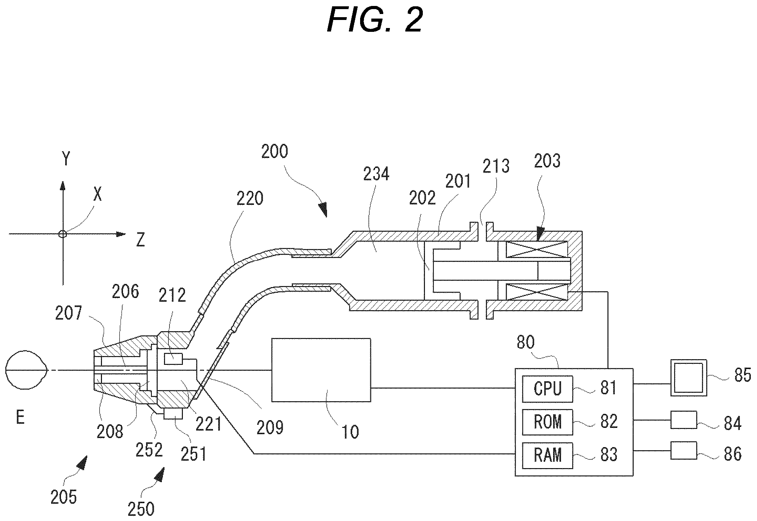

A fluid ejection unit 200 ejects air to the subject eye. As illustrated in FIG. 2, the fluid ejection unit 200 includes, for example, a cylinder 201, a piston 202, a solenoid actuator (hereinafter, also referred to as a solenoid) 203, and the nozzle unit 205. The cylinder 201 and the piston 202 are used as an air compression mechanism for compressing air to be ejected to the subject eye. The cylinder 201 has, for example, a cylindrical shape. The piston 202 slides along an axial direction of the cylinder 201. The piston 202 compresses air in an air compression chamber 234 in the cylinder 201. The solenoid 203 according to the present embodiment is a so-called linear solenoid and operates linearly. The nozzle unit 205 includes, for example, a nozzle 206 and a nozzle holder 207. The nozzle 206 ejects the compressed air to the outside of the apparatus. The nozzle holder 207 accommodates the nozzle 206 therein. The nozzle unit 205 is the approach unit that is disposed in front of the eyes of the examinee during measurement and approaches the examinee.

The air compressed in the air compression chamber 234 in the cylinder 201 by the movement of the piston 202 is ejected from the nozzle 206 toward a cornea of a subject eye E via a tube (which may be a pipe) 220 coupled to a tip of the cylinder 201 and an airtight chamber 221 accommodating the compressed air. For example, the cylinder 201 may be disposed parallel to a horizontal plane (XZ plane), and the piston 202 may be moved horizontally in the cylinder 201 due to the driving of the solenoid 203, thereby compressing air. For example, the cylinder 201 is disposed such that a longitudinal direction thereof is parallel to a horizontal direction, and an inner surface of the cylinder 201 guides the piston 202. Therefore, a moving direction (compression direction) of the piston 202 is the horizontal direction. Each of the above components is disposed on a stage provided in a housing of an apparatus body.

The fluid ejection unit 200 may include, for example, glass plates 208 and a glass plate 209. The glass plate 208 is transparent, holds the nozzle 206, and transmits observation light and alignment light. The glass plate 209 constitutes a rear wall of the airtight chamber 221 and transmits the observation light and the alignment light. An observation/alignment optical system is disposed behind the glass plate 209 so that an observation optical axis and an alignment optical axis thereof are coaxial with an axis of the nozzle 206.

The fluid ejection unit 200 may include, for example, a pressure sensor 212 and an air vent hole 213. The pressure sensor 212 detects, for example, the pressure of the airtight chamber 221. The air vent hole 213 reduces resistance until the piston 202 gains initial speed, for example, and a pressure change that rises proportional to time can be obtained.

The detection unit 250 is, for example, a detection means that detects that the nozzle unit 205 approaches (or comes into contact with) the examinee. The detection unit 250 includes, for example, a contact sensor 251. The contact sensor 251 is, for example, a capacitance sensor. The contact sensor 251 is electrically connected to the nozzle holder 207 by, for example, an electric wire 252. Of course, the contact sensor 251 may be electrically connected to an electrode disposed on a surface of the nozzle holder 207 on an examinee side. The contact sensor 251 may be a pressure-sensitive sensor or the like. In this case, the contact sensor 251 is disposed on a surface of the nozzle unit 205 on the examinee side.

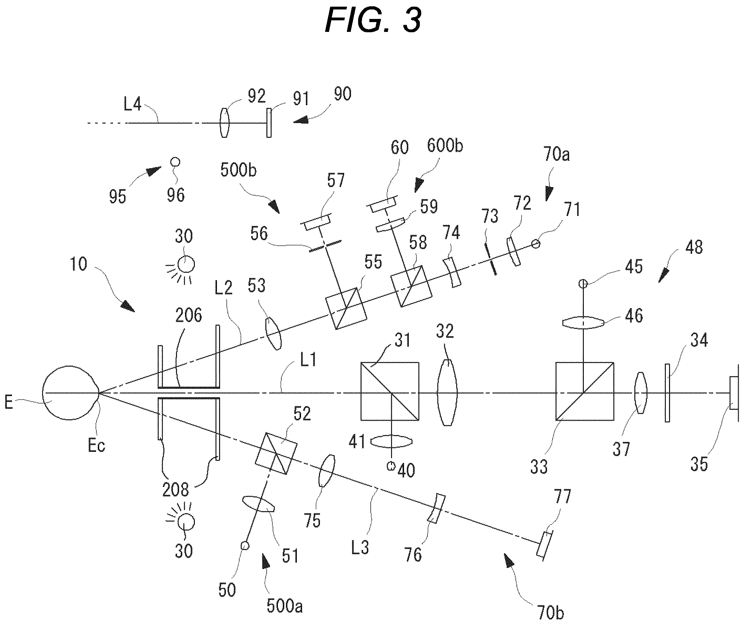

FIG. 3 is a schematic diagram of a measurement optical system 10 of the ophthalmic apparatus 1. A subject eye image illuminated by an infrared illumination light source 30 is formed on a CCD camera 35 via a beam splitter 31, an objective lens 32, a dichroic mirror 33, an imaging lens 37, and a filter 34. That is, the optical system from the beam splitter 31 to the CCD camera 35 has an imaging element and is used as an observation optical system for observing the anterior segment of the subject eye. In this case, an optical axis L1 is used as an observation optical axis.

The filter 34 has a property of transmitting light of the light source 30 and an infrared light source 40 for alignment, and not transmitting visible light and light of a light source 50 for detecting corneal deformation, which will be described later. The image formed on the CCD camera 35 is displayed on a display unit 85.

The infrared light projected from the light source 40 via a projection lens 41 is reflected by the beam splitter 31 and projected onto the subject eye from the front. A cornea bright spot formed at a cornea apex by the light source 40 is imaged on the CCD camera 35 via the beam splitter 31 to the filter 34, and is used for alignment detection in up, down, left, and right directions. That is, the optical system from the beam splitter 31 to the CCD camera 35 has an imaging element and is used as a detection optical system for detecting an alignment state in the up, down, left, and right directions with respect to the subject eye. In this case, the optical axis L1 is used as an alignment optical axis. In the present embodiment, the detection optical system also serves as the observation optical system for observing the anterior segment.

A fixation optical system 48 has the optical axis L1 and presents a fixation target to the subject eye E from a front direction. In this case, the optical axis L1 is used as a fixation optical axis. The fixation optical system 48 includes, for example, a visible light source (fixation lamp) 45, a projection lens 46, and the dichroic mirror 33, and projects light for fixating the subject eye E in the front direction onto the subject eye E. A light source such as an LED or a laser is used as the visible light source 45. As the visible light source 45, for example, a two-dimensional display such as a liquid crystal display is used in addition to a pattern light source such as a point light source, a slit light source, or a ring light source.

Visible light emitted from the visible light source 45 passes through the projection lens 46, is reflected by the dichroic mirror 33, passes through the objective lens 32, and is then projected onto the fundus of the subject eye E. Accordingly, the subject eye E is fixated on a fixation point in the front direction, and a line of sight is fixed. The visible light emitted from the visible light source 45 is converted into a parallel light beam by passing through the projection lens 46 and the objective lens 32.

A corneal deformation detection optical system includes a light projecting optical system 500a and a light receiving optical system 500b, and is used to detect a deformation state of a cornea Ec. Each of the optical systems 500a and 500b is disposed in the examination unit 100 and three-dimensionally moved by a drive unit 4.

The light projecting optical system 500a has an optical axis L3 as a light projecting optical axis, and irradiates the cornea Ec of the subject eye E with illumination light from an oblique direction. The light projecting optical system 500a includes, for example, the light source 50, a collimator lens 51, and a beam splitter 52. The light receiving optical system 500b includes a photodetector 57 and receives reflected light of the illumination light from the cornea Ec of the subject eye E. The light receiving optical system 500b is disposed substantially symmetrically to the light projecting optical system 500a with respect to the optical axis L1. The light receiving optical system 500b includes, for example, a lens 53, a beam splitter 55, a pinhole plate 56, and the photodetector 57, and forms an optical axis L2 as a light receiving optical axis.

Light emitted from the light source 50 is made into a substantially parallel light beam by the collimator lens 51, is reflected by the beam splitter 52, becomes coaxial (coincides) with the optical axis L3 of a light receiving optical system 70b described later, and is projected onto the cornea Ec of the subject eye E. Light reflected by the cornea Ec is coaxial (coincides) with the optical axis L2 of a light projecting optical system 70a described later, passes through the lens 53, is reflected by the beam splitter 55, passes through the pinhole plate 56, and is received by the photodetector 57. The lens 53 is coated with a material that does not transmit light from the light source 30 and the light source 40. The optical system for corneal deformation detection is disposed such that the amount of light received by the photodetector 57 is maximized when the subject eye is in a predetermined deformation state (flat state).

The corneal deformation detection optical system also serves as a part of a first working distance detection optical system, and a light projecting optical system of the first working distance detection optical system also serves as the light projecting optical system 500a of the corneal deformation detection optical system. A light receiving optical system 600b that receives the light reflected by the cornea Ec from the light source 50 includes, for example, the lens 53 of the light receiving optical system 500b, a beam splitter 58, a condenser lens 59, and a position detection element 60, and forms the optical axis L2 as a light receiving optical axis.

Illumination light projected from the light source 50 and reflected by the cornea Ec forms a target image which is a virtual image of the light source 50. The light of the target image passes through the lens 53 and the beam splitter 55, is reflected by the beam splitter 58, passes through the condenser lens 59, and is incident on the one-dimensional or two-dimensional position detection element 60 such as a PSD or a line sensor. Regarding the position detection element 60, when the subject eye E (cornea Ec) moves in a working distance direction (Z direction), the target image formed by the light source 50 also moves on the position detection element 60, so that a control circuit 20 obtains working distance information based on an output signal from the position detection element 60. The output signal from the position detection element 60 according to the present embodiment is used for alignment (rough adjustment) in the working distance direction (Z direction). The light receiving optical system 600b of the first working distance detection optical system does not have a magnification as large as that of the light receiving optical system 70b described below. Therefore, a distance detection range of the position detection element 60 in the Z direction is wider than that of a light receiving element 77.

A corneal thickness measurement optical system includes the light projecting optical system 70a, the light receiving optical system 70b, and the fixation optical system 48, and is used to measure a corneal thickness of the subject eye E. The light projecting optical system 70a also serves as a part of the corneal deformation detection optical system and the first working distance detection optical system.

The light projecting optical system 70a has the optical axis L2 as a light projecting optical axis, and irradiates the cornea Ec of the subject eye E with illumination light (measurement light) from an oblique direction. The light projecting optical system 70a includes, for example, an illumination light source 71, a condenser lens 72, a light restricting member 73, a concave lens 74, and the lens 53 shared with the corneal deformation detection optical system. As the illumination light source 71, a visible light source or an infrared light source (including near-infrared) is used, and for example, a light source such as an LED or a laser is used. The condenser lens 72 condenses light emitted from the illumination light source 71. The light source 50 and the illumination light source 71 use wavelength bands different from each other.

The light restricting member 73 is disposed in an optical path of the light projecting optical system 70a and restricts light emitted from the illumination light source 71. The light restricting member 73 is disposed at a position substantially conjugate with the cornea Ec. The light restricting member 73 may be, for example, a pinhole plate or a slit plate. The light restricting member 73 is used as an aperture that allows a part of light emitted from the illumination light source 71 to pass therethrough and blocks the remaining light. The light projecting optical system 70a forms a predetermined pattern light beam (for example, a spot light beam or a slit light beam) on the cornea Ec of the subject eye E.

The light receiving optical system 70b includes the light receiving element 77 and receives reflected light of the illumination light on a front surface and a back surface of the cornea of the subject eye E. The light receiving optical system 70b is disposed substantially symmetrically to the light projecting optical system 70a with respect to the optical axis L1. The light receiving optical system 70b includes, for example, a light receiving lens 75, a concave lens 76, and the light receiving element 77, and forms the optical axis L3 as a light receiving optical axis. The light receiving optical system 70b illustrated in FIG. 3 also serves as a second working distance detection optical system that detects an alignment state in the Z direction with respect to the subject eye E.

The light receiving element 77 includes a plurality of photoelectric conversion elements, and receives reflected light from the front surface and the back surface of the cornea. As the light receiving element 77, for example, a light detection device such as a one-dimensional line sensor or a two-dimensional area sensor is used. The light receiving optical system 70b of the corneal thickness measurement optical system and the second working distance detection optical system performs observation with a large magnification. Therefore, a distance detection range of the light receiving element 77 in the Z direction is narrower than that of the position detection element 60.

When the subject eye E (cornea Ec) moves in the working distance direction (Z direction), the reflected light of the illumination light source 71 on the cornea Ec also moves on the light receiving element 77, so that the control unit 80 obtains working distance information based on an output signal from the light receiving element 77 of the second working distance detection optical system. Further, the control unit 80 recognizes the corneal deformation state and blinking of the subject eye E based on the output signal from the light receiving element 77, and controls the driving of the solenoid 203.

Light emitted from the illumination light source 71 is condensed by the condenser lens 72 and illuminates the light restricting member 73 from behind. The light from the illumination light source 71 is restricted by the light restricting member 73, and then imaged (condensed) by the lens 53 near the cornea Ec. For example, a pinhole image (when a pinhole plate is used) or a slit image (when a slit plate is used) is formed near the cornea Ec. At this time, the light from the illumination light source 71 forms an image near an intersection with a visual axis on the cornea Ec.

When illumination light is projected onto the cornea Ec by the light projecting optical system 70a, reflected light of the illumination light from the cornea Ec travels in a direction symmetrical to the light projecting light beam with respect to the optical axis L1. The reflected light is imaged on a light receiving surface of the light receiving element 77 by the light receiving lens 75.

The lens 53, which is shared by the light receiving optical systems 500b and 600b and the light projecting optical system 70a, is positioned to condense light reflected by the cornea Ec from the light source 50 on a central portion of the hole of the pinhole plate 56 and condense illumination light from the illumination light source 71 on the front surface and the back surface of the cornea Ec.

A face imaging unit 90 is, for example, an optical system for imaging a face including at least one of the right and left subject eyes. For example, as illustrated in FIG. 3, the face imaging unit 90 according to the present embodiment mainly includes, for example, an imaging element 91 and an imaging lens 92.

The face imaging unit 90 is provided, for example, at a position where both subject eyes can be imaged when the examination unit 100 is at an initial position. In the present embodiment, the initial position of the examination unit 100 is set to a position shifted to the right side with respect to the optical axis L1 of the examination unit 100 so that the right eye is easily examined. Therefore, the face imaging unit 90 is provided at a position where both subject eyes can be imaged in a state where the examination unit 100 is at the initial position shifted to the right side. For example, the face imaging unit 90 is disposed at a machine center in a state where the examination unit 100 is at the initial position. For example, when the initial position is set based on half the pupillary distance, that is, the monocular pupillary distance, the face imaging unit 90 may be disposed at a position shifted to the left or right by the monocular pupillary distance with respect to the machine center of the apparatus body.

The face imaging unit 90 according to the present embodiment is moved together with the examination unit 100 by the drive unit 4. Of course, the face imaging unit 90 may be configured to be fixed to a base 2 and not to move, for example.

The imaging lens 92 may be, for example, a wide-angle lens. The wide-angle lens is, for example, a fisheye lens or a conical lens. By providing the wide-angle lens, the face imaging unit 90 can image the face of the examinee at a wide angle of view.

A face illumination optical system 95 illuminates the face of the subject eye. The face illumination optical system 95 includes, for example, illumination light sources 96. The illumination light source 96 emits infrared light. In the present embodiment, the illumination light sources 96 are provided at left and right sides of an optometry window. The face illumination optical system 95 uses a light source having lower directivity than a target light source for alignment.

As illustrated in FIG. 2, the ophthalmic apparatus 1 includes the control unit 80. The control unit 80 performs various controls of the ophthalmic apparatus 1. The control unit 80 includes, for example, a general CPU (Central Processing Unit) 81, a ROM 82, and a RAM 83. For example, the ROM 82 stores an ophthalmic apparatus control program for controlling the ophthalmic apparatus 1, initial values, and the like. For example, the RAM 83 temporarily stores various types of information. The control unit 80 is connected to the examination unit 100, the face imaging unit 90, the drive unit 4, the display unit 85, an operation unit 86, a jaw rest drive unit 3d, a storage unit (for example, nonvolatile memory) 84, an audio output unit 89, and the like. The storage unit 84 is, for example, a non-transitory storage medium capable of storing storage contents even when a supply of power is cut off. For example, a hard disk drive or a removable USB flash memory may be used as the storage unit 84.

As illustrated in FIG. 1, the ophthalmic apparatus 1 may include the base 2, a face support unit 3, the drive unit 4, the display unit 85, the audio output unit 89, the face imaging unit 90, and the like. The base 2 movably supports the examination unit 100. The face support unit 3 supports the face of the examinee. The face support unit includes a forehead rest 3a, a jaw rest 3b, a jaw rest sensor 3c, and the jaw rest drive unit 3d. The jaw rest sensor 3c detects whether a jaw is placed on the jaw rest 3b. The jaw rest drive unit 3d adjusts the height by moving the jaw rest 3b up and down. The drive unit 4 moves the examination unit 100 in XYZ directions (three-dimensional directions) with respect to the base 2. The display unit 85 displays, for example, an observation image and a measurement result of the subject eye. For example, the display unit 85 may be provided integrally with the ophthalmic apparatus 1 or may be provided separately from the apparatus. The display unit 85 may be disposed such that a display screen faces not only the examinee but also the examinee side. The display unit 85 may be used as the operation unit 86. In this case, the display unit 85 is used for various settings of the ophthalmic apparatus 1 and an operation at the start of measurement. Various operation instructions by the examiner or the examinee are input to the display unit 85. As the operation unit 86, various human interfaces such as a joystick, a mouse, a keyboard, a trackball, and a button may be used. The face imaging unit 90 images, for example, the face of the subject. The audio output unit 89 makes an audio announcement to the examinee or the examiner. The audio output unit 89 is, for example, a speaker. The face imaging unit 90 images, for example, a face including at least one of the left and right subject eyes.

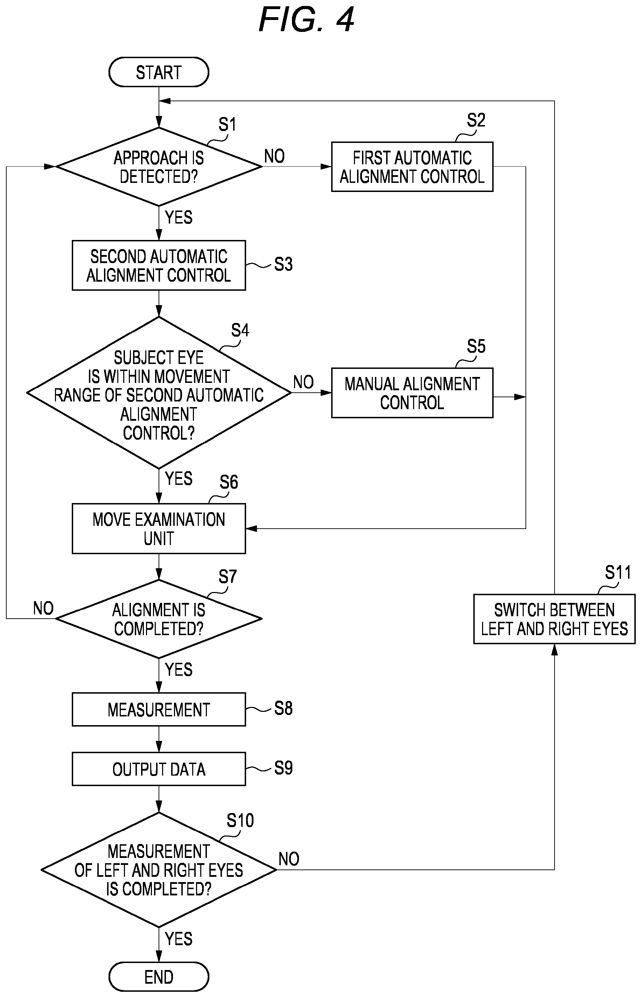

A control operation of the ophthalmic apparatus 1 having the above configuration will be described with reference to FIG. 4. As illustrated in FIG. 4, the ophthalmic apparatus 1 according to the present embodiment has first automatic alignment control and second automatic alignment control. The first automatic alignment control and the second automatic alignment control are alignment control for automatically aligning the nozzle unit 205 with the subject eye E. Assuming that the automatic alignment range in the first automatic alignment control is a first automatic alignment range and the automatic alignment range in the second automatic alignment control is a second automatic alignment range, the second automatic alignment range is set to be smaller than the first automatic alignment range. The control unit 80 switches from the first automatic alignment control to the second automatic alignment control when the detection unit 250 detects the approach of the nozzle unit 205 to the examinee. The ophthalmic apparatus 1 also has manual alignment control. The manual alignment control is alignment control in which the control unit 80 aligns the nozzle unit 205 with the subject eye E by the examiner manually operating the operation unit.

When the face of the examinee is placed on the face support unit 3, the control unit 80 detects that the face is placed on the face support unit 3 by the jaw rest sensor 3c, and detects both subject eyes from a face image captured by the face imaging unit 90. Examples of a method for detecting the subject eyes from the image include various image processing methods such as pupil detection by infrared imaging and edge detection by a luminance value. For example, when infrared imaging is performed on the face of the examinee, the skin appears white and the pupil appears black. Therefore, the control unit 80 may detect a round black (low luminance) part as the pupil from the infrared image obtained by infrared imaging. By using the method as described above, the control unit 80 detects the subject eye E from the face image and acquires two-dimensional position information thereof.

When the subject eye E is detected, the control unit 80 determines whether the detection unit 250 detects the approach to the examinee (S1). When the approach to the examinee is not detected (S1 = NO), the control unit 80 performs the first automatic alignment control. The control unit 80 controls the drive unit 4 to automatically move the examination unit 100 to perform alignment with respect to the subject eye. For example, the control unit 80 moves the examination unit 100 in the direction in which the subject eye is detected in the face image. In addition, the control unit 80 moves the examination unit 100 in the direction in which the subject eye is detected until the subject eye appears in an anterior segment image captured by the observation optical system. When the subject eye appears in the anterior segment image, the control unit 80 performs alignment of the examination unit 100 based on a bright spot appearing in the anterior segment image.

When the approach to the examinee is detected (S1 = YES), the control unit 80 switches the alignment control to the second automatic alignment control (S3). The automatic alignment range of the second automatic alignment control is smaller than that of the first automatic alignment control. The control unit 80 determines whether the subject eye is within the second automatic alignment range (S4). When the subject eye is not within the second automatic alignment range (S4 = NO), the control unit 80 switches the alignment control to the manual alignment control (S5), and moves the examination unit 100 based on the examiner's operation on the operation unit 86 (S6). When the subject eye is within the second automatic alignment range (S4 = YES), the control unit 80 moves the examination unit 100 (S6).

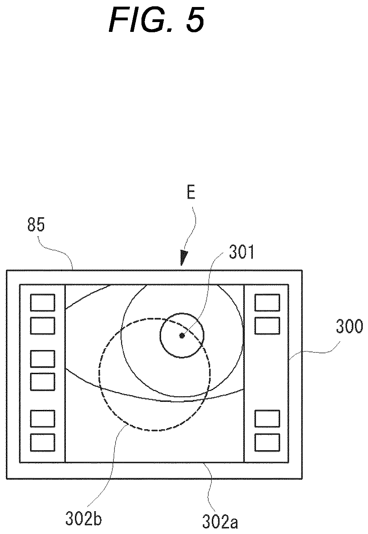

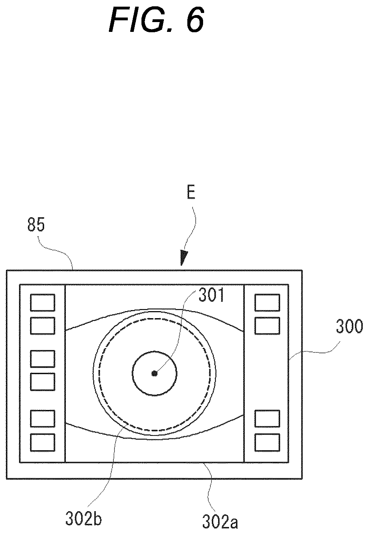

The second automatic alignment range may be set as follows. For example, the first automatic alignment range of the examination unit 100 in the X direction or the Y direction is a range 302a in the entire anterior segment image. In this case, as illustrated in FIG. 5, the second automatic alignment range is a range 302b smaller than the range 302a in the entire anterior segment image. The second automatic alignment range is, for example, a range in which the examination unit 100 is substantially in front of the subject eye. When detecting a bright spot 301 appearing in the anterior segment image in the second automatic alignment range (see FIG. 5), the control unit 80 moves the examination unit 100 to perform alignment (see FIG. 6). Further, for example, when the control unit 80 detects the bright spot 301 appearing in the anterior segment image outside the second automatic alignment range, the control unit 80 switches to the manual alignment control. Accordingly, the measurement can be performed while ensuring safety. Similarly, the second automatic alignment range in the Z direction is set to be smaller than the first automatic alignment range. For example, if the first automatic alignment range is the entire range in which the working distance information can be detected by the working distance detection optical system, the second automatic alignment range is set to a range smaller than the first automatic alignment range.

When the control unit 80 does not receive a signal indicating the detection of the approach from the detection unit 250 during the alignment (S1 = NO), the control unit 80 performs the first automatic alignment control (S2) and moves the examination unit 100 (S6).

After moving the examination unit 100, the control unit 80 determines whether the alignment is completed (S7). When the alignment is not completed (S7 = NO), the processing returns to S1, and the detection unit 250 determines whether the approach of the nozzle unit 205 to the examinee is detected.

When the alignment is completed (S7 = YES), the control unit 80 measures the subject eye (S8). For example, the control unit 80 measures the corneal thickness of the subject eye by the corneal thickness measurement optical system. The control unit 80 calculates a distance (peak-to-peak distance) between a reflection signal from the front surface of the cornea and a reflection signal from the back surface of the cornea, which are detected by the light receiving element.

When the measurement of the corneal thickness is completed, the control unit 80 measures the intraocular pressure. For example, when the control unit 80 drives the solenoid 203 to move the piston 202, air in the cylinder 201 is compressed, and the compressed air is blown from the nozzle 206 toward the cornea Ec. The cornea Ec is gradually deformed due to the blowing of compressed air, and the maximum amount of light is incident on the photodetector 57 when the cornea Ec reaches a flat (or applanation) state. The control unit 80 obtains an intraocular pressure value based on an output signal from the pressure sensor 212 and an output signal from the photodetector 57. Then, the measurement result is displayed on the display unit 85 (S9). Here, when a predetermined measurement end condition is satisfied, the intraocular pressure measurement of the subject eye is completed.

When measuring both eyes, the control unit 80 determines whether the measurement of the left and right eyes is completed. When the measurement of the left and right eyes is not completed (S10 = NO), the control unit 80 moves the examination unit 100 to the eye that is not measured (S11: switch between the left eye and the right eye). For example, after performing the measurement of the right eye, the control unit 80 disposes the examination unit 100 in front of the left eye. Thereafter, the left eye is measured in the same manner as the right eye.

The control unit 80 performs the second automatic alignment control while the detection unit 250 detects the approach to the examinee. When the detection unit 250 does not detect the approach (S1 = NO), the control unit 80 switches the alignment control to the first automatic alignment control.

When the measurement is completed, the control unit 80 outputs data of the measurement result (S9). For example, the control unit 80 displays the measurement result on the display unit 85, prints out the measurement result, or outputs the measurement result to the outside of the apparatus in a wireless or wired manner.

In the above embodiment, when the approach of the nozzle unit 205 to the examinee is detected by the detection unit 250, the control unit 80 performs alignment by narrowing the automatic alignment range of the examination unit 100. However, the control unit 80 may perform the alignment by reducing a moving speed of the examination unit 100. For example, the control unit 80 causes the detection unit 250 to detect the approach of the nozzle unit 205 to the examinee in the first automatic alignment control, and switches the alignment control to the second automatic alignment control. The second automatic alignment control may be automatic alignment control in which a moving speed of the examination unit 100 is reduced as compared with the first automatic alignment control.

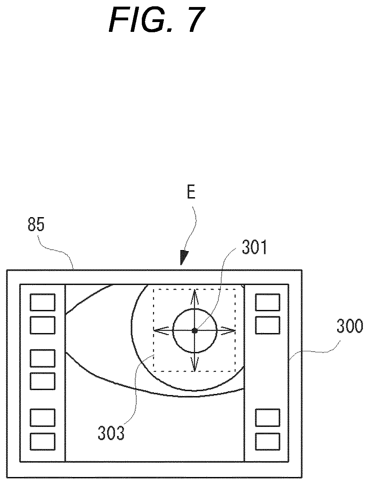

In the above embodiment, the second automatic alignment control is configured such that the control unit 80 performs alignment when detecting the bright spot 301 within the second automatic alignment range of the approach unit in the anterior segment image. However, for example, the control unit 80 may be configured to set a predetermined movement range from a position where the approach of the approach unit to the examinee or a movement distance from the position to the examinee is detected by the detection unit 250, and to restrict the movement until the approach is no longer detected (movement range 303 in FIG. 7).

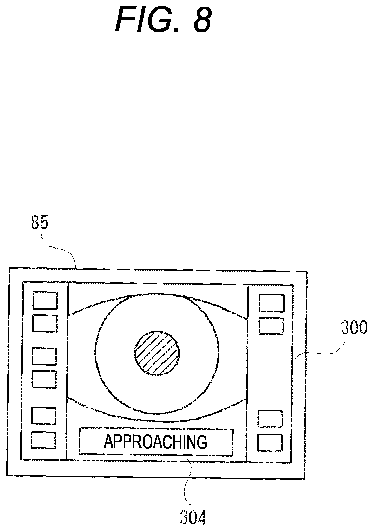

In the above embodiment, while performing the second automatic alignment control, the control unit 80 may notify on a screen 300 of the display unit 85 that the nozzle unit 205 and the examinee are approaching each other (notification 304 in FIG. 8). Instead of displaying the notification 304, the control unit 80 may control the audio output unit 89 to perform the audio announcement, or may perform both the display of the notification 304 and the audio announcement.

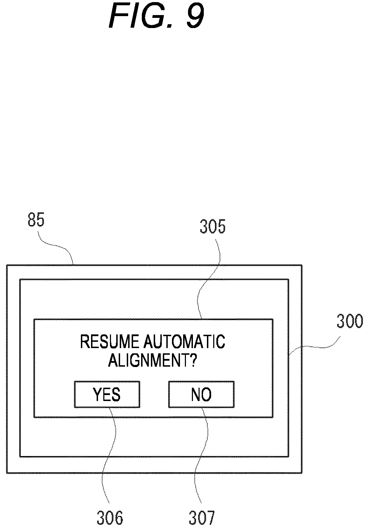

In the above embodiment, when the approach is detected, the control unit 80 continues the automatic alignment without stopping the movement of the apparatus. However, for example, when the approach is detected, the control unit 80 may be configured to temporarily stop the movement of the apparatus. The control unit 80 may display on the screen 300 of the display unit 85 that the approach of the approach unit to the examinee is detected (notification 304 in FIG. 8). In addition, the control unit 80 may display, on a notification 305 notifying the resumption of automatic alignment, a button 306 and a button 307 for indicating that the display content is confirmed (FIG. 9). When the examiner presses the button 306, the control unit 80 may resume the alignment control by the automatic alignment control. When the examiner presses the button 307, the control unit 80 may resume the alignment control based on the examiner operating the operation unit 86 by switching to the manual alignment control. Instead of displaying the notification 304, the audio output unit 89 may be controlled to perform the audio announcement, or both the display of the notification 304 and the audio announcement may be performed.

In the above embodiment, the alignment control of the control unit 80 is set to the second automatic alignment control only while the detection unit 250 detects the approach of the nozzle unit 205 to the examinee. However, the control unit 80 may be configured to switch to the second automatic alignment control once the detection unit 250 detects the approach of the nozzle unit 205 to the examinee, and to continue the second automatic alignment control regardless of whether further approach is detected. However, if the subject eye E is positioned outside the second automatic alignment range during the second automatic alignment control, the control unit 80 may switch the alignment control to the manual alignment control.

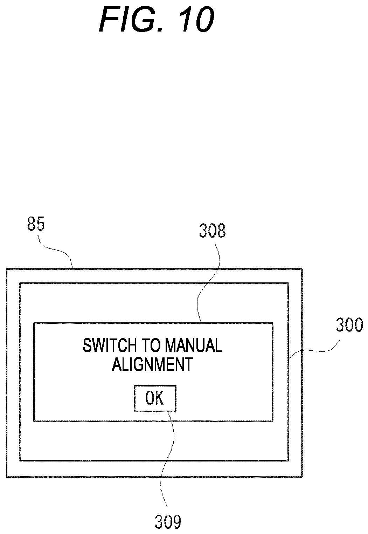

In the above embodiment, when switching to the manual alignment control, the control unit 80 may display on the screen 300 of the display unit 85 that a manual operation is necessary (notification 308 in FIG. 10). In addition, the control unit 80 may display, on the notification 308, a button 309 for indicating that the display content is confirmed. This prevents unintentional (sudden) switching to manual alignment against the examiner's intent. Further, by providing the button 309 on the notification 308, it is possible to confirm whether the examiner recognizes the switch to manual alignment. Instead of displaying the notification 308, the audio output unit 89 may be controlled to perform the audio announcement, or both the display of the notification 308 and the audio announcement may be performed.

The approach unit that approaches the examinee is not limited to the nozzle unit, and may be an examination window (cover glass) of the examination unit 100, an objective lens, an optical attachment, or any other portions having a shape protruding toward the examinee side.

The control unit 80 may cause the ophthalmic apparatus 1 to execute the above processing as illustrated in FIG. 4 stored in the storage unit 84.

## Claims

1. An ophthalmic apparatus for examining a subject eye, the ophthalmic apparatus comprising:
an examination means configured to examine the subject eye;
an approach unit provided with the examination means, the approach unit approaching an examinee;
a detection means configured to detect an approach of the approach unit to the examinee; and
a control means configured to perform, when the approach is detected by the detection means, automatic alignment control different from that performed when the approach is not detected.

2. The ophthalmic apparatus according to claim 1, wherein
in a case where the approach is detected, the control means continues automatic alignment under the automatic alignment control.

3. The ophthalmic apparatus according to claim 1 or 2, wherein
when the approach is detected, the control means performs the automatic alignment control to restrict a movement of the approach unit.

4. The ophthalmic apparatus according to any one of claims 1 to 3, wherein
when the approach is detected, the control means performs the automatic alignment control to reduce an automatic alignment range as compared with when the approach is not detected.

5. The ophthalmic apparatus according to any one of claims 1 to 4, wherein
when the approach is detected, the control means performs the automatic alignment control to reduce a movement range of the approach unit as compared with when the approach is not detected.

6. The ophthalmic apparatus according to any one of claims 1 to 5, wherein
when the approach is detected, the control means performs the automatic alignment control to reduce a moving speed of the approach unit as compared with when the approach is not detected.

7. The ophthalmic apparatus according to any one of claims 1 to 6, wherein
the ophthalmic apparatus is a non-contact tonometer including a fluid discharge means configured to discharge a fluid to a cornea of the subject eye via a nozzle unit, and
the approach unit is the nozzle unit.

8. An ophthalmic apparatus control program of an ophthalmic apparatus for examining a subject eye, the ophthalmic apparatus including an examination means configured to examine the subject eye, an approach unit provided with the examination means, a detection means configured to detect an approach of the approach unit to an examinee, and a control means,
the ophthalmic apparatus control program comprising instructions which, when executed by a control means of the ophthalmic apparatus, cause the ophthalmic apparatus to perform:
a detection step of detecting an approach of the approach unit approaching an examinee by the detection means; and
a control step of performing, when the approach is detected in the detection step, automatic alignment control different from that performed when the approach is not detected.
